# FASCICULE DE BREVET EUROPEEN

(11) **EP 1 133 296 B1**
(45) Date de publication et mention de la délivrance du brevet: **13.08.2003**
(21) Numéro de dépôt: 99956109.5
(22) Date de dépôt: 22.11.1999
(51) Int. Cl.: A61K 31/48, A61P 21/00, A61P 25/00

(54) **NOUVELLE APPLICATION THERAPEUTIQUE DE LA NICERGOLINE**
NEUE THERAPEUTISCHE VERWENDUNG VON NICERGOLINE
NOVEL THERAPEUTIC APPLICATION OF NICERGOLINE

(30) Priorité: 24.11.1998 FR 9814793
(43) Date de publication de la demande: 19.09.2001
(73) Titulaire: Aventis Pharma S.A., 92160 Antony (FR)
(72) Inventeur: DIB, Michel, F-75013 Paris (FR)
(74) Mandataire: Rousseau, Pierrick Edouard
(86) Numéro de dépôt international: FR9902867
(87) Numéro de publication internationale: WO00030643

(56) Documents cités:
- EP-A- 0 602 619
- DE-A- 4 240 798
- O. ELWAN ET AL.: "Ergoloids and Ischaemic Strokes; Efficacy and Mechanism of Action" JOURNAL OF INTERNATIONAL MEDICAL RESEARCH, vol. 23, no. 3, 1995, pages 154-166, XP002112644
- V. ZUCCONI ET AL.: "Clinical Study on Treatment with Nicergoline in a Group of Patients with Hemiplegia in a Post-Acute Phase" MINERVA MEDICA, vol. 65, no. 17, 1974, pages 936-945, XP002112651
- K. TAKAHASHI ET AL.: "Nicergoline inhibits T-type ca2+ channels in rat isolated hippocampal CA1 pyramidal neurones" BRITISH JOURNAL OF PHARMACOLOGY, vol. 100, no. 4, 1990, pages 705-710, XP002112645
- DATABASE WPI Section Ch, Week 9408 Derwent Publications Ltd., London, GB; Class B02, AN 94-061991 XP002112646 & JP 06 016555 A (TANABE SEIYAKU CO), 25 janvier 1994 (1994-01-25)

## Description

La présente invention concerne l'utilisation de la nicergoline pour la préparation d'un médicament destiné à prévention et/ou au traitement des maladies motoneuronales.

La nicergoline ou (8β)-10-méthoxy-1,6-diméthylergoline-8-méthanol-5-bromonicotinate (Sermion®) présente notamment des propriétés α-bloquantes, α2-adrénolytiques (CARPENE C. et coll., J. Pharmacol, 14, 57-66 (1983)), antiischémiques (CAHN R. et coll., Chem. Abstracts, 107, 228784x (1987); UEDAT et coll., Chem. Abstracts, 118, 225224f (1993)), anticalciques (TAKAHASHI K. et coll., Br. J. Pharmacol., 100, 705-710 (1990)), antioxydantes (TANAKA M. et coll., Neurosci. Let., 248, 67-72 (1998)), antithrombotiques, (Chem. Abstracts 105, 54314k (1986)). Elle améliore la capacité d'apprentissage et la mémoire (Chem. Abstracts, 113, 52358u (1990); Chem. Abstracts, 111, 108396h, 1989; Chem. Abstracts, 109, 86208c, 1988; Chem. Abstracts, 106, 12788e, 1987; Chem. Abstracts, 115, 198237s, 1991).

Il a maintenant été trouvé que la nicergoline augmente la survie des motoneurones et peut ainsi être utilisée dans la prévention et/ou le traitement des maladies motoneuronales.

Les maladies motoneuronales incluent la sclérose latérale amyotrophique, l'atrophie musculaire spinale progressive, l'atrophie musculaire infantile, la slérose latérale primaire.

En présence de support trophique fourni par les facteurs neurotrophiques BDNF ou GDNF, les cultures de motoneurones sont composées de neurones larges et homogènes avec de longs neurites branchés. Cependant, les motoneurones meurent par apoptose si la culture est effectuée en absence de support trophique.

L'effet de la nicergoline a donc été déterminé dans un modèle de dégénération induite par privation de facteur neurotrophique de motoneurones en culture.

Par ailleurs, les astrocytes jouent un rôle majeur dans le contrôle et la maintenance d'un environnement adéquat pour la survie motoneuronale.

La nicergoline a ainsi également été testée sur une co-culture de motoneurones et d'astrocytes.

Les protocoles utilisés sont les suivants :

### CULTURES ENRICHIES EN MOTONEURONES :

Les cultures enrichies en motoneurones sont préparées en utilisant la méthode de centrifugation décrite par R.L. SCHNAAR et A.E. SCHAFFNER, J. Neurosci., 1, 204-217 (1981) et modifiée par W. CAMU et C.E. HENDERSON, J. Neurosci. Methods, 44, 59-70 (1992). Sur des plaques de cultures préalablement enduites de laminine/ornithine selon la méthode de A.G. ESTEVEZ et coll., J. Neurosci., 18 (3), 923-931 (1998), on étale les motoneurones à une densité de 2500 cellules par plaque de 35 mm. Les cultures sont ensuite maintenues dans du milieu L15 (GIBCO BRL) contenant du bicarbonate de sodium (22mM), de la conalbumine (0,1 mg/ml), de la putrescine (0,1 mM), de l'insuline (5 µg/ml), du sélénite de sodium (31 nM), du glucose (20 mM), de la progestérone (21 nM), de la pénicilline (100 IU/ml) et de la streptomycine (100 ug/ml).

Les motoneurones ainsi obtenus sont composés de neurones larges (25-30 µM) et homogènes avec de longs neurites branchés. Plus de 70% des cellules sont immunoréactives pour le récepteur neurotrophine p75 et les marqueurs Islet ½ pour les motoneurones spinaux. Environ 70% des motoneurones meurent par apoptose 24 heures après l'étalement si la culture est effectuée en absence de facteur trophique.

### CULTURE D'ASTROCYTES DE MOELLE EPINIERE :

Les astrocytes sont obtenus à partir de jeunes rats âgés d'un jour selon la méthode de R.P. SANETO ET J. DE VELLIS, in Neurochemistry a practical approach (A.J. TURNER et H.S. St JOHN) IRL Press, Oxford-Washington DC, p27-63 légèrement modifiée. Les moëlles épinières sont disséquées stérilement, débarassées des méninges et des ganglions dorsaux. Cinq à dix moëlles épinières sont transferrées dans du PBS (phosphate buffer saline) et coupées avant incubation à 37°C pendant 25 minutes dans du PBS auquel on a ajouté 0,25% de trypsine. Le traitement enzymatique est stoppé par addition de 10 ml de milieu Dubelcco-Eagle modifié (DMEM) auquel on a ajouté 10% de sérum foetal de veau (FCS) et les cellules sont collectées par centrifugation. Une autre étape de dissociation mécanique est effectuée en utilisant l'extrémité d'une pipette de 1 ml. Les cellules sont étalées à une densité de 1,5-2x10⁶ cellules pour 25 cm² de milieu de culture dans du DMEM à 10% de FCS. Après 2 jours in vitro les cultures sont alimentées chaque jour. Quand une monocouche visible de cellules est terminée, les cultures sont agitées 48 heures à 250 rpm et, le lendemain, les monocouches sont traitées par du cytosine arabinoside (10⁻⁵ M) pendant 48 heures. Les monocouches d'astrocytes sont ensuite amplifiées à une densité de cinq sur des plaques de culture de 35mm pour des flacons de culture de 25 cm² au départ.

Les cultures d'astrocytes spinaux sont composées à plus de 98% de cellules polygonales, plates et immunoréactives pour la protéine gliale fibrilaire acide (GFAP). Les monocouches sont exposées au produit à tester et ensuite incubées avec le milieu motoneuronal pour obtenir un milieu de culture conditionné. Ce milieu est transféré et testé à différentes dilutions pour déterminer ses effets sur la survie neuronale.

### IMMUNOCHIMIE

Les cellules sont fixées dans 4% de paraformaldéhyde et 0,1% de glutaraldéhyde dans du PBS (pH 7,4 à 4°C pendant 15 minutes) et dans une solution méthanolique froide. Les cultures sont ensuite lavées et les sites non spécifiques bloqués avec 10% de sérum de chèvre et 2 % d'albumine de sérum bovin (BSA) dans du PBS et traités pour immunochimie en utilisant des anticorps contre le récepteur de neurotrophine de faible affinité p75 ou une protéine de neurofilaments 200 kD (Amersham) en utilisant les instructions du fabricant et en appliquant la réaction d'amplification avidine-biotine DAB/peroxyde d'hydogène.

### TRAITEMENT DES ASTROCYTES AVEC LA NICERGOLINE

Le traitement des astrocytes avec la nicergoline s'effectue de la manière suivante : le produit est dissous dans du méthanol, stérilisé par filtration et utilisé immédiatement après préparation. Le traitement appliqué aux cultures de motoneurones enrichies est effectué par addition d'aliquotes des produits à tester en solution au milieu L15 par étalement. Les monocouches d'astrocytes sont exposées au véhicule ou aux solutions du composé à tester pendant 24 heures et à différentes concentrations. Les monocouches d'astrocytes sont lavées 3 fois avec du DMEM et incubées avec du milieu complet L15. Le milieu conditionné d'astrocytes est repris 24 heures plus tard et centrifugé à 1800 g pendant 15 minutes et utilisé immédiatement ou conservés à - 70°C 2 semaines maximum sans perte d'activité trophique.

### COMPTAGE DES CELLULES ET ANALYSE STATISTIQUE

Les cellules immunoréactives pour les neurofilaments et exhibant des neurites plus longs que les diamètres des cellules sont considérés comme des motoneurones viables. Le nombre de motoneurones est évalué par comptage des cellules marquées dans une surface de 0,4-1 cm² sous microscope grossissant 200 fois. Dans tous les cas, les valeurs sont exprimées comme un pourcentage du nombre de motoneurones présents dans les cultures maintenues avec des facteurs trophiques. Les expériences sont réalisées au moins 3 fois.

Les analyses statistiques sont effectuées en utilisant le test de Student (t-test).

Les résultats obtenus sont les suivants :
1 - Effet de la nicergoline sur la survie motoneuronale en co-cultures astrocytes/motoneurones:

| | % DE MOTONEURONES MARQUES |
|---|---|
| véhicule seul | 100±9,4 |
| nicergoline | |
| 0,1 µM | 115,7±16 |
| 1 µM | 145,5±24* |
| 10 µM | 163,7±22,5** |

| | |
|---|---|
| * significativement différent du véhicule (p<0,05) | |
| ** significativement différent du véhicule (p<0,01) | |

ND non déterminé
Ces résultats démontrent que la nicergoline à la concentration de 10 µM augmente la survie motoneuronale de 63,7% en comparaison aux co-cultures traitées avec le véhicule seul.
2 - effet neurotrophique-like de la nicergoline sur la mort neuronale dans des cultures enrichies en motoneurones et en absence de facteur trophique :

Dans ce test, la nicergoline augmente la survie des motoneurones de 13 % (p<0,05).

La présente invention concerne également l'utilisation de la nicergoline pour la préparation d'un médicament destiné au traitement des maladies motoneuronales et notamment la sclérose latérale amyotrophique, l'atrophie musculaire spinale progressive, l'atrophie musculaire infantile, la sclérose latérale primaire.

La nicergoline peut être préparée selon le brevet US3228943.

Les médicaments sont constitués par au moins la nicergoline, à l'état pur ou sous forme d'une composition dans laquelle il est associé à tout autre produit pharmaceutiquement compatible, pouvant être inerte ou physiologiquement actif. Les médicaments selon l'invention peuvent être employés notamment par voie orale ou parentérale.

Comme compositions solides pour administration orale, peuvent être utilisés des comprimés, des pilules, des poudres (capsules de gélatine, cachets), des lyophilisants oraux (Lyoc^{R}) ou des granulés. Dans ces compositions, le principe actif est mélangé à un ou plusieurs diluants inertes, tels que amidon, acide tartrique, gomme arabique, saccharine sodique, vanilline, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

### EXEMPLES DE FORMULATION :

gélules : 5 mg de nicergoline et comme exipients du talc, du lactose et du stéarate de magnésium
lyophilisat oral : 5 mg de nicergoline et comme excipients de l'acide tartrique, du lactose, de la gomme arabique, de la saccharine sodique et de la vanilline
poudre pour usage parentéral : 5 mg de nicergoline et comme excipients de l'acide tartrique et du lactose.

Les doses dépendent de l'effet recherché, de la durée du traitement et de la voie d'administration utilisée; elles sont généralement comprises entre 30 et 100 mg par jour par voie orale pour un adulte avec des doses unitaires allant de 5 à 20 mg de substance active.

D'une façon générale, le médecin déterminera la posologie appropriée en fonction du poids et de tous les autres facteurs propres au sujet à traiter.

## Revendications

1. Utilisation de la nicergoline pour la préparation d'un médicament destiné à la prévention et/ou au traitement des maladies motoneuronales.

2. Utilisation selon la revendication 1 pour le traitement de la sclérose latérale amyotrophique, l'atrophie musculaire spinale progressive, l'atrophie musculaire infantile, la sclérose latérale primaire.

3. Utilisation selon l'une des revendications 1 ou 2 pour la préparation d'un médicament contenant 5 à 20 mg de nicergoline.

## Patentansprüche

1. Verwendung von Nicergoline zur Herstellung eines Arzneimittels, das zur Prävention und Behandlung von motoneuronalen Erkrankungen bestimmt ist.

2. Verwendung nach Anspruch 1 zur Behandlung amyotropher Lateralsklerose, progressiver spinaler Muskelatrophie, Kindermuskelatrophie, primärer Lateralsklerose.

3. Verwendung nach Anspruch 1 oder Anspruch 2 zur Herstellung eines Arzneimittels, das 5 bis 20 mg Nicergoline enthält.

## Claims

1. Use of nicergoline for preparing a pharmaceutical which is intended for preventing and/or treating motor neuron diseases.

2. Use according to Claim 1 for treating amyotrophic lateral sclerosis, progressive spinal muscular atrophy, infantile muscular atrophy and primary lateral sclerosis.

3. Use according to either of Claims 1 and 2 for preparing a pharmaceutical comprising from 5 to 20 mg of nicergoline.
